# FASCICULE DE BREVET EUROPEEN

(11) **EP 1 570 836 B1**
(45) Date de publication et mention de la délivrance du brevet: **26.03.2008**
(21) Numéro de dépôt: 05290360.6
(22) Date de dépôt: 17.02.2005
(51) Int. Cl.: A61Q 19/04, A61K 8/35, A61K 8/84

(54) **Gel autobronzant transparent contenant un polymère d'acide acrylamido 2-méthyl propane sulfonique hydrosoluble ou hydrodispersible**
Selbstbräunendes durchsichtiges Gel enthaltend einen wasserlöslichen oder wasserdispergierbaren Polymer aus Acrylamido-2-methylpropylsulfonsäure
Transparent self-tanning gel comprising a hydrosoluble or hydrodispersible polymer of acrylamido 2-methyl propane sulfonic acid

(30) Priorité: 04.03.2004 FR 0450439
(43) Date de publication de la demande: 07.09.2005
(73) Titulaire: L'ORÉAL, 75008 Paris (FR)
(72) Inventeur: Josso Martin, Paris (FR)
(74) Mandataire: Miszputen, Laurent

(56) Documents cités:
- EP-A- 0 576 189
- EP-A- 0 604 249
- EP-A- 0 715 845
- EP-A- 0 829 258
- EP-A- 1 013 266
- WO-A-93/07856
- FR-A- 2 816 316
- FR-A- 2 819 183
- US-B2- 6 613 755

## Description

L'invention concerne des gels aqueux transparents à usage topique destinées au bronzage et/ou au brunissage artificiels de la peau, caractérisées par le fait qu'elle comprennent, dans un support cosmétiquement acceptable, au moins un agent autobronzant mono- ou polycarbonylé et au moins un polymère ou copolymère particulier, hydrosoluble ou hydrodispersible, réticulé ou non-réticulé comportant au moins le monomère acide acrylamido 2-méthyl propane sulfonique (AMPS), sous forme partiellement ou totalement neutralisée par la soude.

L'invention porte également sur un procédé de traitement cosmétique pour bronzer et/ou brunir artificiellement la peau, caractérisé par le fait qu'il consiste à appliquer sur celle-ci une quantité efficace d'une telle composition.

On sait que les composés mono- ou polycarbonylés tels que par exemple l'isatine, l'alloxane, la ninhydrine, le glycéraldéhyde, l'aldéhyde mésotartrique, la glutaraldéhyde, l'érythrulose, la dihydroxyacétone (DHA) sont des produits particulièrement intéressants qui sont couramment utilisés en cosmétique comme agents de bronzage artificiel de la peau.

Appliqués sur la peau, notamment sur le visage, ces composés permettent d'obtenir un effet de bronzage ou de brunissage d'apparence plus ou moins semblable à celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

Les formules transparentes cosmétiques sont de plus en plus appréciées par le consommateur pour des raisons esthétiques. Les gels aqueux sont particulièrement recherchés en cosmétique du fait de leur apport en eau conférant une sensation agréable de fraîcheur sur la peau et du fait qu'ils ne confèrent pas un aspect gras.

On peut obtenir des gels aqueux transparents à base de polymères acryliques réticulés du type Carbomer comme les produits Carbopol de Noveon, mais ces gélifiants sont incompatibles avec les agents autobronzants comme la dihydroxyacétone (DHA). On peut également réaliser des formulations aqueuses épaissies à base d'hydroxyethylcellulose et d'hydroxypropylguar (Jaguar HP de Rhodia). Les produits obtenus ont un toucher filant peu agréable, sont très collants après application, et présentent une stabilité dans le temps médiocre. L'utilisation comme agent gélifiant de dérivés cellulosiques donnent des gels transparents qui ont tendance à pelucher à l'application.

On connaît également des gels aqueux autobronzants contenant un gélifiant du type polyacrylamide dans la demande WO93/07856 notamment l'exemple II comprenant de la dihydroxyacétone, le mélange Polyacrylamide/C₁₃-C₁₄ Isoparaffine/Laureth-7 et de l'alcool benzylique. Ce type de composition de permet pas d'obtenir des gels transparents.

On connaît également dans la demande FR 2 816 316 un exemple de gel autobronzant transparent contenant le polymère Ammonium Polyacryloyldimethyl Taurate (polymère réticulé d'acide acrylamido 2-méthyl propane sulfonique partiellement neutralisé par l'ammoniaque vendu sous le nom commercial Hostacerin AMPS) en association avec un copolymère d'anhydride/alkylvinyléther (STABILEZE QM). Ce type de gel n'est pas stable dans le temps.

On connaît également dans les demandes EP0604249 et EP576189 des formulations autobronzantes à base de dihydroxyacétone et d'un copolymère réticulé d'acide acrylamido 2-méthyl propane sulfonique/acrylamide sous forme d'émulsion. On a proposé également dans la demande FR2819183 d'utiliser dans des produits autobronzants des copolymères d'acide acrylamido 2-méthyl propane sulfonique amphiphiles comprenant une partie hydrophile et une partie hydrophobe comportant au moins une chaîne grasse.

Il existe donc le besoin de rechercher de nouvelles compositions autobronzantes à base d'un composé mono- ou polycarbonylé sous forme de gel aqueux transparent qui ne présentent pas les inconvénients définis ci-dessus et qui présentent de bonnes propriétés cosmétiques : toucher non filant, non peluchant, effet frais, une bonne stabilité dans le temps et présentent une bonne efficacité auto-bronzante sur la peau (intensité et tenue de la coloration).

Ainsi, à la suite d'importantes recherches menées sur la question, il a maintenant été trouvé par la Demanderesse, et ceci de façon tout à fait inattendue et surprenante, qu'il est possible d'atteindre ces objectifs en utilisant un polymère ou copolymère, hydrosoluble ou hydrodispersible, réticulé ou non-réticulé comportant au moins le monomère acide acrylamido 2-méthyl propane sulfonique (AMPS), sous forme libre ou bien partiellement ou totalement neutralisée.

Cette découverte est à la base de la présente invention.

L'invention concerne donc un gel aqueux transparent autobronzant, caractérisé par le fait qu'il comprend, dans un support cosmétiquement acceptable, au moins un agent autobronzant mono- ou polycarbonylé et au moins un polymère ou copolymère, hydrosoluble ou hydrodispersible, réticulé ou non-réticulé comportant au moins le monomère acide acrylamido 2-méthyl propane sulfonique (AMPS), sous forme partiellement ou totalement neutralisée par la soude ledit polymère ou copolymère d'AMPS étant choisi dans le groupe constitué par :
(i) les homopolymères d' AMPS, réticulés ou non réticulés ;
(ii) les copolymères d'AMPS et d'hydroxyéthyl acrylate.

Au sens de la présente invention, on entendra, par «coloration artificielle de la peau », une coloration non couvrante (non opacifiant) et durable qui ne s'élimine ni à l'eau ni à l'aide d'un solvant, et qui résiste à la fois au frottement et au lavage par une solution contenant des tensioactifs. Une telle coloration non couvrante durable se distingue donc de la coloration couvrante et momentanée apportée par exemple par un produit de maquillage.

L'invention porte également sur un procédé de traitement cosmétique pour bronzer et/ou brunir artificiellement la peau, caractérisé par le fait qu'il consiste à appliquer sur celle-ci une quantité efficace d'une telle composition.

On entend par "gel aqueux" , une composition contenant une phase aqueuse continue contenant une masse visco-élastique formée à partir de suspensions colloïdales. La viscosité d'un gel selon l'invention est mesurée à 25°C, avec un appareil RM180 RHEOMAT (mobile 2 ou 3) de la société Rheometric Scientific et sa valeur est en général d'au moins 60 UD au mobile 2 (Unités de Déviation)

On entend par "transparent " une composition présentant une turbidité inférieure à 400 NTU (Unités de Turbidité Nephélométique) à 25°C et de préférence inférieure à 250 NTU à 25°C, mesurée avec un appareil 2100P Turbidimeter de la société HACH.

Les gels conformes à la présente invention comprennent une phase aqueuse en général dans une proportion supérieure ou égale à 70% en poids et de préférence supérieure ou égale à 80% en poids et plus particulièrement supérieure ou égale à 90% en poids par rapport au poids total du gel.

Les polymères utilisés conformément à l'invention sont des homopolymères ou copolymères, réticulés ou non-réticulés comportant au moins le monomère acide acrylamido 2-méthyl propane sulfonique (AMPS), sous forme partiellement ou totalement neutralisée par la soude .

Ils sont de préférence neutralisés totalement ou pratiquement totalement neutralisés, c'est-à-dire neutralisés à au moins 90 %.

Ces polymères d'AMPS selon l'invention peuvent être réticulés ou non-réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Les polymères d'AMPS conformes à l'invention sont hydrosolubles ou hydrodispersibles. Ils sont dans ce cas :
- soit des "homopolymères" ne comportant que des monomères AMPS et, s'ils sont réticulés, un ou plusieurs agents de réticulation tels que ceux définis ci-dessus ;
- soit des copolymères obtenus à partir de l'AMPS et d'hydroxyethyl acrylate

Par « hydrosoluble ou hydrodispersible », on entend des polymères qui, introduits dans une phase aqueuse à 25°C, à une concentration massique égale à 1%, permettent l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 60%, de préférence d'au moins 70%.

Les "homopolymères" selon l'invention sont de préférence réticulés et neutralisés, et ils peuvent être obtenus selon le procédé de préparation comprenant les étapes suivantes :
(a) on disperse ou on dissout le monomère tel que l'AMPS sous forme libre dans une solution de tertio-butanol ou d'eau et de tertio-butanol ;
(b) on neutralise la solution ou la dispersion de monomère obtenue en (a) , dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

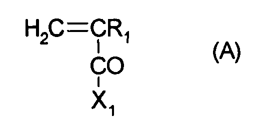

Les polymères d'AMPS hydrosolubles ou hydrodispersibles de l'invention ont de préférence une masse molaire allant de 50 000 g/mole à 10 000 000 g/mole, de préférence de 80 000 g/mole à 8 000 000 g/mole, et de façon encore plus préférée de 100 000 g/mole à 7 000 000 g/mole.

Comme homopolymères hydrosolubles ou hydrodispersibles d'AMPS conformes à l'invention, on peut citer par exemple les polymères réticulés ou non d' acrylamido-2-methyl propane sulfonate de sodium tel que celui utilisé dans le produit commercial SIMULGEL 800 (nom CTFA : Sodium Polyacryloyldimethyl Taurate).

Comme copolymères hydrosolubles ou hydrodispersibles d'AMPS conformes à l'invention, on peut citer par exemple
- les copolymères d'AMPS et d'hydroxyéthyl acrylate, comme par exemple le copolymère AMPS/hydroxyéthyl acrylate tel que celui utilisé dans le produit commercial vendu sous la dénomination SIMULGEL NS par la société SEPPIC (nom CTFA : Hydroxyethyl acrylate/Sodium Acryloyldimethyltaurate copolymer (and) Squalane (and) Polysorbate 60).

Les polymères préférés sont plus particulièrement les homopolymères acrylamido-2-methyl propane sulfonate de sodium tels que celui utilisé dans le produit commercial SEPIGEL 800 et les copolymère AMPS/hydroxyéthyl acrylate tels que celui utilisé dans le produit commercial vendu sous la dénomination SIMULGEL NS

Selon une forme particulièrement préférée de l'invention, on utilisera le polymère ou copolymère d'AMPS sous forme de poudre.

Les polymères d'AMPS conformes à l'invention sont généralement présents dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids de par rapport au poids total du gel.

Les agents autobronzants mono ou polycarbonylés sont choisis par exemple parmi l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrits dans la demande de brevet FR 2466492 et WO 9735842, la dihydroxyacétone (DHA), les dérivés de 4,4-dihydroxypyrazolin-5-ones telles que décrits dans la demande de brevet EP-A-0903342.

Dans un mode de réalisation particulièrement préféré de l'invention on utilisera plus particulièrement comme agent autobronzant la dihydroxyacétone (DHA).

Les agents autobronzants conformes à l'invention sont généralement présents dans les compositions dans des proportions allant de 0,1 à 10% en poids par rapport au poids total de la composition, et de préférence de 0,2 à 8% en poids par rapport au poids total de la composition

Les compositions conformes à l'invention peuvent contenir en plus d'autres agents de coloration artificielle de la peau parmi lesquels on peut citer notamment :
(i) les dérivés indoliques comme les monohydroxyindoles tels que décrits dans le brevet FR2651126 ( ie : 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole) ;
(ii) les extraits végétaux agents de coloration artificielle de la peau tels que :
   - les extraits de bois rouges "insolubles" du genre Pterocarpus et du genre Baphia comme le Pterocarpus santalinus, le Pterocarpus osun, le Pterocarpus soyauxii, le Pterocarpus erinaceus, le Pterocarpus indicus ou encore le Baphia nitida tels que ceux décrits dans la demande de brevet EP-A-0 971683 ;
   - les extraits végétaux du genre Saxifraga comme le Saxifraga cuneifolia, Saxifraga glaucescens, Saxifraga rotundifolia, Saxifraga granulata, Saxifraga bulbifera, Saxifraga umbrosa, Saxifraga tridactylites ;
   - les extraits végétaux du genre Sorgho comme le Sorghum caudatum , le Sorghum Bicolor.
(iii) les colorants rouges ou oranges du type fluorane comme
   - le tétrabromofluoroscéine ou éosine (CI 45380 ou Red 21)
   - la phloxine B (CI 45410 ou Red 27)
   - la diiodofluorescéine (CI 45425 ou Orange 10) ;
   - la dibromofluorescéine (Cl 45370 ou Orange 5)
   - le sel de sodium de la tétrabromofluoroscéine (Cl 45380 (Na salt) ou Red 22)
   - le sel de sodium de la phloxine B (Cl 45410 (Na salt) ou Red 28)
   - le sel de sodium de la düodofluorescéine (CI 45425 (Na salt) ou Orange 11) ;
   - l'érythrosine (CI 45430 ou Acid Red 51).
   - la phloxine (Cl 45405 ou Acid Red 98).
(iv) le caramel.

Les compositions peuvent également contenir un peptisant pour favoriser l'obtention de la transparence de la composition. On peut citer notamment le decylglucosine, vendu sous le nom « ORAMIX CG 110 » par SEPPIC, l'huile de ricin hydrogénée oxyethylénée vendue sous le nom « CREMOPHOR RH 60 » et « CREMOPHOR RH 40 » par BASF

Les compositions peuvent également contenir en plus au moins un filtre UV organique actif dans l'UVA et/ou l'UVB ; ledit filtre UV pouvant être hydrosoluble, liposoluble ou bien insoluble dans les solvants cosmétiques couramment utilisés.

Les filtres organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les dérivés de méthylène bis-(hydroxyphényl benzotriazole) tels que décrits dans s les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649, les 4,4-diarylbutadiènes tels que ceux décrits dans les demandes de brevet DE19755649 , EP916335, EP1133980, EP1133981 et EP-A-1008586 et leurs mélanges.

Comme exemples de filtres organiques actifs dans l'UV-A et/ou l'UV-B, on peut citer désignés ci-dessus sous leur nom INCI :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

### Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11» par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12
- le 2-(4-diéthylamino-2-hydroxybenzoyl)-benzoate de n-hexyle

### Dérivé du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MESORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

### Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

### Dérivés du phenyl benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés du benzalmalonate :

- Polyorganosiloxane à fonctions benzalmalonate vendu sous la dénomination commerciale « PARSOL SLX » par HOFFMANN LA ROCHE

### Dérivés de 4,4-diarylbutadiène

1,1-dicarboxy-(2'2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres UV organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 2-(4-diéthylamino-2-hydroxybenzoyl)-benzoate de n-hexyle
- 4-Methylbenzylidene camphor,
- Benzimidazilate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- Drometrizole Trisiloxane,
- Polysilicone 15
- 1,1-dicarboxy-(2'2'-diméthyl-propyl)-4,4-diphénylbutadiène
- 2,4-bis-[5-1 (diméthylpropyl)benzoxazol-2-yl-(4-phenyl)-imino]-6-(2-ethylhexyl)-imino-1,3,5-triazine et leurs mélanges.
- et leurs mélanges.
   Les filtres UV conformes à l'invention sont généralement présents dans les gels selon l'invention dans des proportions allant de 0,1 à 20% en poids et de préférence allant de 0,2 à 15% en poids par rapport au poids total du gel.
   Les gels conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti-radicaux libres, les stabilisants, des sequestrants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique et/ou dermatologique, en particulier pour la fabrication de compositions sous forme de gels aqueux transparents.
   Les corps gras peuvent être constitués par une huile ou des mélanges d'huile. Par huile, on entend un composé liquide à température ambiante.
   Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.
   La phase aqueuse desdites compositions contient de l'eau et en général d'autres solvants solubles ou miscibles dans l'eau. Les solvants solubles ou miscibles dans l'eau comprennent les mono alcools à chaîne courte par exemple en C₁-C₄ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.
   Bien entendu, l'homme de l'art veillera à choisir le ou les éventuels composés complémentaires cités ci-dessus et/ou leurs quantités de manière telle que les propriétés avantageuses attachées intrinsèquement à l'invention ne soient pas, ou substantiellement pas, altérées par la ou les adjonctions envisagées.
   L'invention concerne également l'utilisation de ce gel aqueux pour la fabrication d'un produit de coloration artificielle de la peau.
   L'invention concerne également un procédé de traitement cosmétique pour bronzer et/ou brunir artificiellement la peau, caractérisé par le fait qu'il consiste à appliquer sur celle-ci une quantité efficace d'une composition telle que définie ci-dessus.
   Des exemples concrets, mais nullement limitatifs, illustrant l'invention, vont maintenant être donnés.

### EXEMPLES

Dans tous les exemples ci-dessous,
- la viscosité a été mesurée à 25°C avec un appareil RM180 RHEOMAT de la société Rheometric Scientific au mobile 3; elle est exprimée en UD (Unités de Déviation).
- la turbidité a été mesurée à 25°C avec un appareil 2100P Turbidimeter de la société HACH ; elle est exprimée en NTU (Unités de Turbidité Néphélométrique).

### Exemple 1: Gel autobronzant transparent

- Dihydroxyacétone 5,0% en poids
- Decylglucoside (Oramix CG 110) 1,5% en poids
- Sodium Polyacryloyldimethyl Taurate en poudre (MP 6123 de SEPPIC, dérivé du SIMULGEL 800) 1,0% en poids
- Eau purifiée 92,5% en poids
   Turbidité 5 NTU à 25°C
   Viscosité 40 UD (mobile 3) à 25°C
   pH 5

### Exemple 2: Gel autobronzant transparent

- Dihydroxyacétone 5,0% en poids
- Decylglucoside (Oramix CG 110) 1,5% en poids
- Copolymère Hydroxyethylacrylate/ Sodium Acryloyldimethyl Taurate en poudre ( MP 5955 de SEPPIC, dérivé du SIMULGEL NS) 1,0% en poids
- Eau purifiée 92,5% en poids
Turbidité 5 NTU à 25°C
Viscosité 25 UD (mobile 3) à 25°C
pH 5

### Exemple 3: Gel autobronzant transparent

- Dihydroxyacétone 5,0% en poids
- Huile de ricin hydrogénée oxyethylénée (60 OE) (Cremophor RH 60 de BASF 1,5% en poids
- Sodium Polyacryloyldimethyl Taurate en poudre (MP 6123 de SEPPIC, dérivé du SIMULGEL 800) 1,0% en poids
- Eau purifiée 92,5% en poids
Turbidité 87 NTU à 25°C
Viscosité 53 UD (mobile 3) à 25°C
pH 5

### Exemple 4: Gel autobronzant transparent

- Dihydroxyacétone 5,0% en poids
- Huile de ricin hydrogénée oxyethylénée (60 OE) (Cremophor RH 60 de BASF 2,0% en poids
- Sodium Polyacryloyldimethyl Taurate en poudre (MP 6123 de SEPPIC, dérivé du SIMULGEL 800) 1,0% en poids
- DL-Alpha-Tocopherol (Vitamine E) 0,1% en poids
- Glycerol 5,0% en poids
- Propylène Glycol 5,0% en poids
- parfum 0,3% en poids
- Eau purifiée 81,6% en poids
Turbidité 218 NTU à 25°C
Viscosité 58 UD (mobile 3) à 25°C
pH 5,4

### Exemple 5 (COMPARATIF)

**Gel autobronzant opaque selon le document** WO9307856 **exemple II page 12**
- Dihydroxyacétone 3,0% en poids
- Polyacrylamide/C₁₃-C₁₄ Isoparaffin/ laureth-7 ( SEPIGEL 305 de SEPPIC) 3,0% en poids
- Alcool benzylique 0.5% en poids
- Eau purifiée 93,5% en poids
Turbidité > 1000 NTU à 25°C
Viscosité 90 UD (mobile 3) à 25°C
pH 5,8
Le gel obtenu n'est pas transparent.

## Revendications

1. Gel aqueux transparent autobronzant ayant une turbidité inférieure à 400 NTU, **caractérisé par le fait qu'**il comprend, dans un support cosmétiquement acceptable, au moins un agent autobronzant mono- ou polycarbonylé et au moins un polymère
ou copolymère, hydrosoluble ou hydrodispersible, réticulé ou non-réticulé comportant au moins le monomère acide acrylamido 2-méthyl propane sulfonique (AMPS), sous forme partiellement ou totalement neutralisée par la soude ; ledit polymère ou copolymère d'AMPS étant choisi dans le groupe constitué par :
**(i)** les homopolymères d' AMPS, réticulés ou non réticulés ;
**(ii)** les copolymères d'AMPS et d'hydroxyéthyl acrylate.

2. Gel selon la revendication 1, **caractérisé en ce qu'**il comprend une phase aqueuse dans une proportion supérieure ou égale à 70% en poids et de préférence supérieure ou égale à 80% en poids et plus particulièrement supérieure ou égale à 90% poids par rapport au poids total du gel.

3. Gel selon l'une quelconque des revendications 1 et 2, où le polymère d'AMPS est neutralisé à au moins 90 %.

4. Composition selon l'une quelconque des revendications 1 à 3, où le polymère d'AMPS est réticulé et où l'agent de réticulation est choisi parmi les composés à polyinsaturation oléfinique couramment utilisés pour la réticulation des polymères obtenus par polymérisation radicalaire.

5. Composition selon la revendication 4, où l'agent de réticulation est choisi parmi le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou leurs mélanges.

6. Composition selon la revendication 5, où l'agent de réticulation est choisi parmi le méthylène-bis-acrylamide, le méthacrylate d'allyle ou le triméthylol propane triacrylate (TMPTA).

7. Gel selon l'une quelconque des revendications 1 à 6, où le taux de réticulation va en général de 0,01 à 10 % en mole et plus particulièrement de 0,2 à 2 % en mole par rapport au polymère.

8. Gel selon l'une quelconque des revendications 1 à 7, où les polymères d'AMPS hydrosolubles ou hydrodispersibles ont une masse molaire allant de 50 000 g/mole à 10 000 000 g/mole, de préférence de 80 000 g/mole à 8 000 000 g/mole, et de façon encore plus préférée de 100 000 g/mole à 7 000 000 g/mole.

9. Gel selon l'une quelconque des revendications 1 à 8 où les homopolymères d' AMPS hydrosolubles ou hydrodispersibles sont choisis parmi les polymères acrylamido-2-methyl propane sulfonate de sodium

10. Gel selon l'une quelconque des revendications 1 à 9, **caractérisé par le fait que** le polymère ou le copolymère d'AMPS est sous forme de poudre.

11. Gel selon l'une quelconque des revendications 1 à 10, **caractérisé par le fait que** le polymère ou le copolymère d'AMPS est présent dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids de par rapport au poids total du gel.

12. Gel selon l'une quelconque des revendications 1 à 11 , **caractérisé par le fait que** les agents autobronzants mono ou polycarbonylés sont choisis parmi l'isatine, l'alloxane, la ninhydrine, le glycéraldéhyde, l'aldéhyde mésotartrique, la glutaraldéhyde, l'érythrulose, les dérivés de pyrazolin-4,5-diones, la dihydroxyacétone (DHA), les dérivés de 4,4-dihydroxypyrazolin-5-ones.

13. Gel selon la revendication 12, où l'agent autobronzant est la dihydroxyacétone (DHA).

14. Gel selon l'une quelconque des revendications 1 à 13 , **caractérisé par le fait que** les agents autobronzants sont présents dans des proportions allant de 0,1 à 10% en poids et de préférence de 0,2 à 8% en poids par rapport au poids total du gel.

15. Gel selon l'une quelconque des revendications 1 à 14, **caractérisé par le fait qu'**il contient en plus d'autres agents de coloration artificielle de la peau.

16. Gel selon l'une quelconque des revendications 1 à 15 , **caractérisé par le fait qu'**il contient en plus un peptisant.

17. Gel selon la revendication 16 , où le peptisant est choisi parmi le décylglucosine et l'huile de ricin hydrogénée oxyethylénée.

18. Gel selon l'une quelconque des revendications 1 à 17 , **caractérisé par le fait qu'**il contient en plus au moins un filtre UV organique actif dans l'UVA et/ou l'UVB.

19. Gel selon la revendication 18 , où les filtres organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de benzoxazole ; les dérivés de méthylène bis-(hydroxyphényl benzotriazole) ; les polymères filtres et silicones filtres ; les dimères dérivés d'α-alkylstyrène ; les 4,4-diarylbutadiènes et leurs mélanges.

20. Gel selon la revendication **19** , où les filtres organiques sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 2-(4-diéthylamino-2-hydroxybenzoyl)-benzoate de n-hexyle
- 4-Methylbenzylidene camphor,
- Benzimidazilate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- Drometrizole Trisiloxane,
- Polysilicone 15
- 1,1-dicarboxy-(2'2'-diméthyl-propyl)-4,4-diphénylbutadiène
- 2,4-bis-[5-1 (diméthylpropyl)benzoxazol-2-yl-(4-phenyl)-imino]-6-(2-ethylhexyl)-imino-1,3,5-triazine et leurs mélanges.
- et leurs mélanges.

21. Gel selon l'une quelconque des revendications 18 à 20 où les filtres organiques sont présents dans les compositions selon l'invention dans des proportions allant de 0,1 à 20% en poids par rapport au poids total de la composition, et de préférence allant de 0,2 à 15% en poids par rapport au poids total de la composition.

22. Composition selon l'une quelconque des revendications 1 à 21, **caractérisée par le fait qu'**elle comprend en outre des adjuvants cosmétiques classiques choisis parmi les corps gras, les solvants organiques, les épaississants ioniques ou non ioniques, les adoucissants, les antioxydants, les agents anti-radicaux libres, les stabilisants, des sequestrants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

23. Procédé de traitement cosmétique pour bronzer et/ou brunir artificiellement la peau, **caractérisé par le fait qu'**il consiste à appliquer sur celle-ci une quantité efficace d'un gel tel que défini dans l'une quelconque des revendications précédentes.

## Claims

1. Self-tanning transparent aqueous gel having a turbidity of less than 400 NTU, **characterized in that** it comprises, in a cosmetically acceptable support, at least one monocarbonyl or polycarbonyl self-tanning agent and at least one water-soluble or water-dispersible, crosslinked or non-crosslinked polymer or copolymer comprising at least the acrylamido-2-methylpropanesulfonic acid (AMPS) monomer, in a form partially or totally neutralized with sodium hydroxide; the said AMPS polymer or copolymer being chosen from the group constituted of:
(i) crosslinked or non-crosslinked AMPS homopolymers;
(ii) copolymers of AMPS and of hydroxyethyl acrylate.

2. Gel according to Claim 1, **characterized in that** it comprises an aqueous phase in a proportion of greater than or equal to 70% by weight, preferably greater than or equal to 80% by weight and more particularly greater than or equal to 90% by weight relative to the total weight of the gel.

3. Gel according to either of Claims 1 and 2, in which the AMPS polymer is at least 90% neutralized.

4. Composition according to any one of Claims 1 to 3, in which the AMPS polymer is crosslinked and in which the crosslinking agent is chosen from the polyolefinically unsaturated compounds commonly used for the crosslinking of polymers obtained by free-radical polymerization.

5. Composition according to Claim 4, in which the crosslinking agent is chosen from divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allylic or vinyl ethers of polyfunctional alcohols, and also allylic esters of phosphoric and/or vinylphosphonic acid derivatives, or mixtures thereof.

6. Composition according to Claim 5, in which the crosslinking agent is chosen from methylenebisacrylamide, allyl methacrylate and trimethylolpropane triacrylate (TMPTA).

7. Gel according to any one of Claims 1 to 6, in which the degree of crosslinking generally ranges from 0.01 mol% to 10 mol% and more particularly from 0.2 mol% to 2 mol% relative to the polymer.

8. Gel according to any one of Claims 1 to 7, in which the water-soluble or water-dispersible AMPS polymers have a molar mass ranging from 50 000 g/mol to 10 000 000 g/mol, preferably from 80 000 g/mol to 8 000 000 g/mol and even more preferably from 100 000 g/mol to 7 000 000 g/mol.

9. Gel according to any one of Claims 1 to 8, in which the water-soluble or water-dispersible AMPS homopolymers are chosen from sodium acrylamido-2-methylpropanesulfonate polymers.

10. Gel according to any one of Claims 1 to 9,
**characterized in that** the AMPS polymer or copolymer is in powder form.

11. Gel according to any one of Claims 1 to 10, **characterized in that** the AMPS polymer or copolymer is present in active material amounts ranging from 0.01% to 20% by weight, more preferably from 0.1% to 10% by weight, even more preferably from 0.1% to 5% by weight and even more particularly from 0.5% to 2% by weight relative to the total weight of the gel.

12. Gel according to any one of Claims 1 to 11, **characterized in that** the monocarbonyl or polycarbonyl self-tanning agents are chosen from isatin, alloxan, ninhydrin, glyceraldehyde, mesotartaric aldehyde, glutaraldehyde, erythrulose, pyrazoline-4,5-dione derivatives, dihydroxyacetone (DHA) and 4,4-dihydroxypyrazolin-5-one derivatives.

13. Gel according to Claim 12, in which the self-tanning agent is dihydroxyacetone (DHA).

14. Gel according to any one of Claims 1 to 13, **characterized in that** the self-tanning agents are present in proportions ranging from 0.1% to 10% by weight and preferably from 0.2% to 8% by weight relative to the total weight of the gel.

15. Gel according to any one of Claims 1 to 14, **characterized in that** it also contains other agents for artificially colouring the skin.

16. Gel according to any one of Claims 1 to 15, **characterized in that** it also contains a peptizer.

17. Gel according to Claim 16, in which the peptizer is chosen from decylglucosine and hydrogenated oxyethylenated castor oil.

18. Gel according to any one of Claims 1 to 17, **characterized in that** it additionally contains at least one UVA-active and/or UVB-active organic UV-screening agent.

19. Gel according to Claim 18, in which the organic screening agents are chosen from anthranilates; cinnamic derivatives; dibenzoylmethane derivatives; salicylic derivatives; camphor derivatives; triazine derivatives; benzophenone derivatives; β,β-diphenylacrylate derivatives; benzotriazole derivatives; benzalmalonate derivatives; benzimidazole derivatives; imidazolines; bis-benzazolyl derivatives; p-aminobenzoic acid (PABA) derivatives; benzoxazole derivatives; methylenebis(hydroxyphenylbenzotriazole) derivatives; screening polymers and screening silicones; dimers derived from α-alkylstyrene; 4,4-diarylbutadienes, and mixtures thereof.

20. Gel according to Claim 19, in which the organic screening agents are chosen from the following compounds:
- Ethylhexyl salicylate,
- Butyl methoxydibenzoylmethane,
- Ethylhexyl methoxycinnamate,
- Octocrylene,
- Phenylbenzimidazolesulfonic acid,
- Terephthalylidenedicamphorsulfonic acid,
- Benzophenone-3,
- Benzophenone-4,
- Benzophenone-5,
- n-Hexyl 2-(4-diethylamino-2-hydroxybenzoyl)benzoate,
- 4-Methylbenzylidenecamphor,
- Benzimidazilate,
- Anisotriazine,
- Ethylhexyltriazone,
- Diethylhexylbutamidotriazone,
- 2,4,6-Tris(diisobutyl 4'-aminobenzalmalonate)-s-triazine,
- Methylenebis(benzotriazolyl)tetramethylbutylphenol,
- Drometrizole trisiloxane,
- Polysilicone-15
- 1,1-Dicarboxy(2',2'-dimethylpropyl)-4,4-diphenylbutadiene,
- 2,4-Bis[5-1(dimethylpropyl)benzoxazol-2-yl(4-phenyl)imino]-6-(2-ethylhexyl)imino-1,3,5-triazine
- and mixtures thereof.

21. Gel according to any one of Claims 18 to 20, in which the organic screening agents are present in the compositions according to the invention in proportions ranging from 0.1% to 20% by weight relative to the total weight of the composition and preferably ranging from 0.2% to 15% by weight relative to the total weight of the composition.

22. Composition according to any one of Claims 1 to 21, **characterized in that** it also comprises standard cosmetic adjuvants chosen from fatty substances, organic solvents, ionic or nonionic thickeners, softeners, antioxidants, free-radical scavengers, stabilizers, sequestrants, emollients, silicones, α-hydroxy acids, antifoams, moisturizers, vitamins, insect repellents, fragrances, preserving agents, surfactants, anti-inflammatory agents, substance P antagonists, fillers, polymers, propellants, acidifying or basifying agents and dyes.

23. Cosmetic treatment process for artificially tanning and/or browning the skin, **characterized in that** it consists in applying thereto an effective amount of a gel as defined in any one of the preceding claims.

## Patentansprüche

1. Selbstbräunendes transparentes wässriges Gel mit einer Trübung unter 400 NTU, **dadurch gekennzeichnet, dass** es in einem kosmetisch akzeptablen Träger mindestens ein Mono- oder Polycarbonyl-Selbstbräunungsmittel und mindestens ein wasserlösliches oder in Wasser dispergierbares, vernetztes oder nicht vernetztes Polymer oder Copolymer enthält, das zumindest das Monomer Acrylamido-2-methylpropansulfonsäure (AMPS) enthält, das mit Natriumhydroxid ganz oder teilweise neutralisiert ist; wobei das Polymer oder Copolymer von AMPS ausgewählt ist unter:
(i) vernetzten oder nicht vernetzten Homopolymeren von AMPS;
(ii) Copolymeren von AMPS und Hydroxyethylacrylat.

2. Gel nach Anspruch 1, **dadurch gekennzeichnet, dass** es eine wässrige Phase in einer Menge von 70 Gew.-% oder darüber und vorzugsweise mindestens 80 Gew.-% und insbesondere mindestens 90 Gew.-%, bezogen auf das Gesamtgewicht des Gels, enthält.

3. Gel nach einem der Ansprüche 1 und 2, wobei das AMPS-Polymer zu mindestens 90 % neutralisiert ist.

4. Zusammensetzung nach einem der Ansprüche 1 bis 3, wobei das AMPS-Polymer vernetzt ist und das Vernetzungsmittel unter den Verbindungen mit mehreren olefinisch ungesättigten Bindungen ausgewählt ist, die gewöhnlich für die Vernetzung von durch radikalische Polymerisation hergestellten Polymeren verwendet werden.

5. Zusammensetzung nach Anspruch 4, wobei das Vernetzungsmittel unter Divinylbenzol, Diallylether, Dipropylenglycoldiallylether, Polyglycoldiallylethern, Triethylenglycoldivinylether, Hydrochinondiallylether, Ethylenglycoldi(meth)acrylat oder Tetra-ethylenglycoldi(meth)acrylat, Trimethylolpropantriacrylat, Methylen-bis-acrylamid, Methylen-bis-methacrylamid, Triallylamin, Triallylcyanurat, Diallylmaleat, Tetraallylethylendiamin, Tetraallyloxyethan, Trimethylolpropan-diallylether, Allyl(meth)-acrylat, Allylethern von Alkoholen der Zuckergruppe oder weiteren Allyl- oder Vinylethern von mehrwertigen Alkoholen sowie Allylestern von Phosphorsäurederivaten und/oder Vinylphosphonsäurederivaten oder deren Gemischen ausgewählt ist.

6. Zusammensetzung nach Anspruch 5, wobei das Vernetzungsmittel unter Methylen-bis-acrylamid, Allylmethacrlyat oder Trimethylolpropantriacrylat (TMPTA) ausgewählt ist.

7. Gel nach einem der Ansprüche 1 bis 6, wobei der Vernetzungsgrad im Allgemeinen im Bereich von 0,01 bis 10 Mol-% und insbesondere 0,2 bis 2 Mol-%, bezogen auf das Polymer, liegt.

8. Gel nach einem der Ansprüche 1 bis 7, wobei die wasserlöslichen oder in Wasser dispergierbaren AMPS-Polymere eine Molmasse von 50 000 bis 10 000 000 g/mol, vorzugsweise 80 000 bis 8 000 000 g/mol und noch bevorzugter 100 000 bis 7 000 000 g/mol aufweisen.

9. Gel nach einem der Ansprüche 1 bis 8, wobei die wasserlöslichen oder in Wasser dispergierbaren AMPS-Homopolymere unter den Polymeren von Natrium-acrylamido-2-methylpropansulfonat ausgewählt sind.

10. Gel nach einem der Ansprüche 1 bis 9, **dadurch gekennzeichnet, dass** das Polymer oder Copolymer von AMPS in Pulverform vorliegt.

11. Gel nach einem der Ansprüche 1 bis 10, **dadurch gekennzeichnet, dass** das Polymer oder Copolymer von AMPS in Wirkstoffmengen von 0,01 bis 20 Gew.-%, vorzugsweise 0,1 bis 10 Gew.-%, noch bevorzugter 0,1 bis 5 Gew.-% und insbesondere 0,5 bis 2 Gew.-%, bezogen auf das Gesamtgewicht des Gels, enthalten ist.

12. Gel nach einem der Ansprüche 1 bis 11, **dadurch gekennzeichnet, dass** die Mono- oder Polycarbonyl-Selbstbräunungsmittel unter Isatin, Alloxan, Ninhydrin, Glyceraldehyd, Mesoweinsäurealdehyd, Glutaraldehyd, Erythrulose, Pyrazolin-4,5-dionderivaten, Dihydroxyaceton (DHA) und 4,4-Dihydroxypyrazolin-5-on-Derivaten ausgewählt sind.

13. Gel nach Anspruch 12, wobei das Selbstbräunungsmittel das Dihydroxyaceton (DHA) ist.

14. Gel nach einem der Ansprüche 1 bis 13, **dadurch gekennzeichnet, dass** die Selbstbräunungsmittel in Mengenanteilen von 0,1 bis 10 Gew.-% und vorzugsweise 0,2 bis 8 Gew.-%, bezogen auf das Gesamtgewicht des Gels, enthalten sind.

15. Gel nach einem der Ansprüche 1 bis 14, **dadurch gekennzeichnet, dass** sie ferner weitere Stoffe zur künstlichen Färbung der Haut enthält.

16. Gel nach einem der Ansprüche 1 bis 15, **dadurch gekennzeichnet, dass** sie ferner ein Peptisierungsmittel enthält.

17. Gel nach Anspruch 16, wobei das Peptisierungsmittel unter Decylglucosin und ethoxyliertem hydrierten Ricinusöl ausgewählt ist.

18. Gel nach einem der Ansprüche 1 bis 17, **dadurch gekennzeichnet, dass** sie ferner mindestens ein im UV-A- und/oder UV-B-Bereich wirksames organisches UV-Filter enthält.

19. Gel nach Anspruch 18, wobei die organischen Filter unter Anthranilaten; Zimtsäurederivaten; Dibenzoylmethanderivaten; Salicylsäurederivaten; Campherderivaten; Triazinderivaten; Benzophenonderivaten; β,β-Diphenylacrylatderivaten; Benzotriazolderivaten; Benzalmalonatderivaten; Benzimidazolderivaten; Imidazolinen; Bis-benzoazolylderivaten; Derivaten von p-Aminobenzoesäure (PABA); Benzoxazolderivaten; Methylen-bis(hydroxyphenylbenzotriazol)-derivaten; Filterpolymeren und Siliconfiltern; von α-Alkylstyrol abgeleiteten Dimeren; 4,4-Diarylbutadienen und deren Gemischen ausgewählt sind.

20. Gel nach Anspruch 19, wobei die organischen Filter unter den folgenden Verbindungen ausgewählt sind:
- Ethylhexyl Salicylate;
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- n-Hexyl-2-(4-diethylamino-2-hydroxybenzoyl)-benzoat,
- 4-Methylbenzylidene Camphor,
- Benzimidazilate,
- Anisotriazine,
- Ethylhexyl Triazone,
- Diethylhexyl Butamido Triazone,
- 2,4,6-Tris(diisobutyl-4'aminobenzalmalonat)-s-triazin,
- Methylene bis-Benzotriazolyl Tetramethylbutylphenol,
- Drometrizole Trisiloxane,
- Polysilicone 15,
- 1,1-Dicarboxy-(2',2'-dimethylpropyl)-4,4-diphenylbutadien,
- 2,4-Bis[5-1-(dimethylpropyl)-benzoxazol-2-yl-(4-phenyl)-imino]-6-(2-ethylhexyl)-imino-1,3,5-triazin und deren Gemische,
- und deren Gemischen.

21. Gel nach einem der Ansprüche 18 bis 20, wobei die organischen Filter in den erfindungsgemäßen Zusammensetzungen in Mengenanteilen von 0,1 bis 20 Gew.-%, bezogen auf das Gesamtgewicht der Zusammensetzung, und vorzugsweise im Bereich von 0,2 bis 15 Gew.-%, bezogen auf das Gesamtgewicht der Zusammensetzung, enthalten sind.

22. Zusammensetzung nach einem der Ansprüche 1 bis 21, **dadurch gekennzeichnet, dass** sie ferner herkömmliche kosmetische Zusatzstoffe enthält, die unter den Fettsubstanzen, organischen Lösungsmitteln, ionischen oder nichtionischen Verdickungsmitteln, beruhigenden Stoffen, Antioxidantien, Radikalfängern für freie Radikale, Stabilisatoren, Maskierungsmitteln, Emollientien, Siliconen, α-Hydroxysäuren, Schaumverhütungsmitteln, Hydratisierungsmitteln, Vitaminen, insektenabwehrenden Wirkstoffen, Parfums, Konservierungsmitteln, grenzflächenaktiven Stoffen, entzündungshemmenden Wirkstoffen, Substanz P-Antagonisten, Füllstoffen, Polymeren, Treibmitteln, Alkalisierungsmitteln oder Ansäuerungsmitteln, Farbmitteln ausgewählt sind.

23. Verfahren zur kosmetischen Behandlung zur Bräunung und/oder künstlichen Braunfärbung der Haut, **dadurch gekennzeichnet, dass** es darin besteht, eine wirksame Menge eines Gels, wie es in einem der vorhergehenden Ansprüche definiert ist, auf die Haut aufzutragen.
